# EUROPEAN PATENT APPLICATION

(11) **EP 4 636 014 A1**
(43) Date of publication of application: **22.10.2025**
(21) Application number: 25170552.1
(22) Date of filing: 15.04.2025
(51) Int. Cl.: C08J 3/12, A61Q 1/00, A61Q 19/00, B01J 2/04, C08L 67/02, C08L 67/04

(54) **RESIN PARTICLE AND METHOD FOR PRODUCING RESIN PARTICLE**

(30) Priority: 18.04.2024 JP 2024067738; 04.02.2025 JP 2025016543
(71) Applicant: Ricoh Company, Ltd., Tokyo 143-8555 (JP)
(72) Inventor: SATO, Yuichi, Yokohama, 220-0011 (JP); INOUE, Ryota, Yokohama, 220-0011 (JP); MORITANI, Tatsuru, Tokyo, 143-8555 (JP)
(74) Representative: Marks & Clerk LLP

(57) **Abstract**

A resin particle is provided that includes a biodegradable resin. The resin particle has a volume average particle size of 5 µm or more and 50 µm or less and a relative span factor of 1.2 or less.

## Description

### BACKGROUND

### Technical Field

The present disclosure relates to a resin particle and a method for producing a resin particle.

### Related Art

A resin particle is used to modify and improve various materials by utilizing its large specific surface area and its particulate structure. Examples of the major applications include a use in a formulation for a cosmetic such as a foundation, an antiperspirant, or a scrubbing agent, a use in various agents such as a matting agent for coating, a rheology modifying agent, an anti-blocking agent, a smoothing agent, a light-diffusing agent, and an agent for medical diagnosis and examination, and a use in an additive for a molded product such as an automotive material or a construction material. Examples of the resin particle include, but are not limited to, a urethane particle, an acrylic particle, a silicone particle, and a polyethylene particle.

In this regard, as concerns over environmental issues have been growing in recent years, there is demand for using a material derived from a non-petroleum raw material or a biodegradable material in any field where a resin is used to reduce the environmental impact. For example, such demand exists in the field such as a cosmetic or coating, where a resin particle is used.

To date, methods for producing a biodegradable resin particle have been proposed, such as a crushing method represented by freeze crushing (WO2008/047863), a solvent dissolution precipitation method in which a resin is dissolved in a solvent at high temperature and cooled to cause precipitation, or a resin is dissolved in a solvent and then precipitated by adding a poor solvent (Japanese Unexamined Patent Application Publication No.2021-147330 and Japanese Unexamined Patent Application Publication No.2005-2302), and a method in which a resin is emulsified at high temperature using a solvent that does not dissolve the resin and a large amount of emulsifier (WO2017/195642).

However, when used in an external agent such as a cosmetic, the resin particle of WO2008/047863 has an issue such as not being spherical or not having a small particle size, requiring further improvement in spreadability on the skin. Further, although the resin particles obtained in Japanese Unexamined Patent Application Publication No.2021-147330, Japanese Unexamined Patent Application Publication No.2005-2302, and WO2017/195642 are relatively spherical, they are insufficient in terms of adhesion to the skin and soft spreadability on the skin, requiring further improvement. The soft spreadability on the skin refers to soft touch and spreadability on the skin.

This soft spreadability on the skin is thought to be related to the softness of the particles, the rolling properties of the particles due to their circularity, the smooth feeling of the powder due to the uniformity of the powder particle size, and the like.

### SUMMARY

An object of the present disclosure described herein is to provide a resin particle that includes a biodegradable resin and is excellent in an adhesion to the skin and a soft use feeling on the skin as a cosmetic.

Embodiments of the present invention provides a resin particle that includes a biodegradable resin. The resin particle has a volume average particle size of 5 µm or more and 50 µm or less and a relative span factor of 1.2 or less.

According to one aspect of the present disclosure, a resin particle that includes a biodegradable resin and is excellent in an adhesion to the skin and a soft use feeling on the skin as a cosmetic is provided.

### BRIEF DESCRIPTION OF THE DRAWINGS

A more complete appreciation of embodiments of the present disclosure and many of the attendant advantages and features thereof can be readily obtained and understood from the following detailed description with reference to the accompanying drawings, wherein:
FIG. 1 is a diagram illustrating particle size distributions of particles produced by a method according to embodiments of the present disclosure and particles produced by a spray drying method;
FIG. 2 is a schematic cross-sectional diagram illustrating a liquid column resonance droplet ejector;
FIG. 3 is a schematic diagram illustrating a resin particle production apparatus;
FIG. 4 is a schematic cross-sectional diagram illustrating a droplet ejector used in the resin particle production apparatus of FIG. 3;
FIG. 5 is a schematic cross-sectional diagram illustrating a droplet ejector used in the resin particle production apparatus of FIG. 3; and
FIG. 6 is a schematic cross-sectional diagram illustrating a Rayleigh fission droplet ejector.

The accompanying drawings are intended to depict embodiments of the present disclosure and should not be interpreted to limit the scope thereof. The accompanying drawings are not to be considered as drawn to scale unless explicitly noted. Also, identical or similar reference numerals designate identical or similar components throughout the several views.

### DETAILED DESCRIPTION

In describing embodiments illustrated in the drawings, specific terminology is employed for the sake of clarity. However, the disclosure of this specification is not intended to be limited to the specific terminology so selected and it is to be understood that each specific element includes all technical equivalents that have a similar function, operate in a similar manner, and achieve a similar result.

Referring now to the drawings, embodiments of the present disclosure are described below. As used herein, the singular forms "a," "an," and "the" are intended to include the plural forms as well, unless the context clearly indicates otherwise.

The inventors of the present invention have studied a resin particle for a cosmetic and have obtained the following findings.

In the conventional technology, the characteristics of particles are determined using an index such as a volume average particle size or a weight average particle size. The inventors of the present invention have found that a resin particle including a biodegradable resin can be excellent in an adhesion to the skin and a soft use feeling on the skin as a cosmetic when the volume average particle size is adjusted to 5 µm or more and 50 µm or less and the relative span factor (R.S.F) is adjusted to 1.2 or less.

The resin particle according to embodiments of the present invention is described in more detail below.

### (Resin particle)

A resin particle according to embodiments of the present invention includes a biodegradable resin as a base material and further includes other components as necessary. Examples of the other components include, but are not limited to, a biologically active substance and a dispersion stabilizer.

The resin particle according to embodiments of the present invention is excellent in an adhesion to the skin and a soft use feeling on the skin, and therefore can be suitably used as a resin particle for a cosmetic. For example, a foundation can be produced by mixing the resin particle according to embodiments of the present invention with a pigment, an oily base material, an emulsifier, a preservative, a fragrance, and the like.

### <Particle size distribution>

The resin particle according to embodiments of the present invention has a relative span factor (R.S.F) of 1.2 or less.

In the present disclosure, the term "relative span factor (R.S.F)" is defined as (D90-D10)/D50 and is an index representing the narrowness of particle size distribution. D90 represents the cumulative 90% by number from the small particle side of the cumulative particle size distribution, D50 represents the cumulative 50% by number from the small particle side of the cumulative particle size distribution, and D10 represents the cumulative 10% by number from the small particle side of the cumulative particle size distribution.

The smaller the R.S.F value, the narrower the particle size distribution.

Examples of a method for measuring the R.S.F include, but are not limited to, a dynamic light scattering method that uses a concentrated system analyzer ("FPAR-1000", manufactured by Otsuka Electronics Co., Ltd.) for measurement and a laser diffraction/scattering method that uses a particle size distribution measuring device ("LA-960", manufactured by Horiba, Ltd.).

Examples of other indexes representing the narrowness of the particle size distribution include "volume average particle size (Dv)/number average particle size (Dn)", which is a value obtained by dividing the volume average particle size (Dv) by the number average particle size (Dn). The smaller the value, the narrower the particle size distribution. The value of "volume average particle size (Dv)/number average particle size (Dn)" of the resin particle according to embodiments of the present invention is preferably 1.00 or more and 1.50 or less, more preferably 1.00 or more and 1.20 or less.

Examples of a method for measuring the volume average particle size (Dv) and the number average particle size (Dn) include, but are not limited to, a measurement method using a laser diffraction/scattering particle size distribution measuring device (device name: Microtrac MT3000 II, manufactured by MicrotracBEL Corp.) and a laser diffraction/scattering method that uses a particle size distribution measuring device ("LA-960", manufactured by Horiba, Ltd.).

### <Volume average particle size>

The volume average particle size (Dv) of the resin particle according to embodiments of the present invention is 5 µm or more and 50 µm or less. From the viewpoint of the touch feeling and the soft spreadability on the skin, it is more preferably 10 µm or more and 30 µm or less.

### <Average circularity>

An average circularity (E) of the resin particle according to embodiments of the present invention is preferably greater than 0.980. From the viewpoint of the touch feeling and the soft spreadability on the skin, it is more preferably 0.985 or more.

The average circularity can be measured, for example, by using a flow type particle image analyzer (product name "FPIA (registered trademark)-3000S", manufactured by Sysmex Corp.).

As the specific measurement method, 0.2 g of the particle group to be measured is added to 20 ml of a 0.25% aqueous solution of sodium dodecylbenzenesulfonate, and ultrasonic waves are applied to the particle group for 5 minutes using an ultrasonic cleaner "MCD-10" manufactured by AS ONE Corp. as a disperser to disperse the particle group in the aqueous surfactant solution, thereby preparing a dispersion liquid for measurement. For the measurement, the above-mentioned flow type particle image analyzer equipped with a standard objective lens (10x) is used, and as a sheath liquid to be used in the flow type particle image analyzer, a particle sheath (product name "PSE-900A", manufactured by Sysmex Corp.) is used. The dispersion liquid for measurement prepared according to the above-mentioned procedure is introduced into the above-mentioned flow type particle image analyzer to perform the measurement under the following measurement conditions. For the measurement, before starting the measurement, the above-mentioned flow type particle image analyzer is subjected to automatic focus adjustment using a suspension liquid of standard polymer particles (e.g., standard polystyrene particles "5200A" manufactured by Thermo Fisher Scientific diluted with ion-exchanged water). Note that the circularity is a value obtained by dividing the perimeter calculated from the diameter of a perfect circle having the same projected area as that of a particle in the captured image by the perimeter of the particle in the captured image.
Measurement mode: HPF mode
Particle size measurement range: 0.996 to 200 µm
Particle circularity measurement range: 0.5 to 1.0
Number of particles measured: 1000

### <Biodegradable resin>

The biodegradable resin is not particularly limited as long as it is a resin having biodegradability. However, the biodegradable resin is preferably at least one resin selected from the group consisting of a polyester resin and a polyamide resin. Examples of the biodegradable polyester resin include, but are not limited to, polylactic acid; poly-ε-caprolactone; a succinate polymer (i.e., polylactic acid-glycolic acid copolymer) such as polyethylene succinate, polybutylene succinate, and polybutylene succinate adipate; polybutylene adipate terephthalate; a polyhydroxyalkanoate such as polyhydroxypropionate, polyhydroxybutyrate, and polyhydroxyvalerate; and polyglycolic acid. Examples of the biodegradable polyamide resin include, but are not limited to, nylon 4. These resins may be used alone or in combination of two or more.

The weight-average molecular weight of the biodegradable resin is preferably 500,000 or less, more preferably 50,000 or less.

### <<Polylactic acid>>

The weight-average molecular weight Mw of the polylactic acid is not particularly limited and can be selected appropriately depending on the purpose. However, it is preferably 5,000 or more and 100,000 or less, more preferably 10,000 or more and 70,000 or less, even more preferably 10,000 or more and 50,000 or less, particularly preferably 40,000 or more and 50,000 or less.

### <<Polyglycolic acid>>

The polyglycolic acid is not particularly limited and can be appropriately selected depending on the purpose. Examples thereof include, but are not limited to, a lactic acid-glycolic acid copolymer, which is a copolymer having a structural unit derived from lactic acid and a structural unit derived from glycolic acid, a glycolic acid-caprolactone copolymer, which is a copolymer having a structural unit derived from glycolic acid and a structural unit derived from caprolactone, and a glycolic acid-trimethylene carbonate copolymer, which is a copolymer having a structural unit derived from glycolic acid and a structural unit derived from trimethylene carbonate. These may be used alone or in combination of two or more. Of these, a lactic acid-glycolic acid copolymer is preferable because it is highly biocompatible, can slowly release the physiologically active substance included in the particle, and can store the physiologically active substance included in the particle for a long period of time.

Examples of the lactic acid-glycolic acid copolymer that can be used include, but are not limited to, PURASORB PDLG5010, PURASORB PDLG7510, PURASORB PDLG7507, PURASORB PDLG5002A, PURASORB PDLG5002, and PURASORB PDLG7502A (manufactured by Corbion), and RG502, RG502H, RG503, RG503H, RG504, and RG504H (manufactured by Sigma-Aldrich).

The weight-average molecular weight of the lactic acid-glycolic acid copolymer is not particularly limited and can be appropriately selected depending on the purpose. However, it is preferably 2,000 to 250,000, more preferably 2,000 to 100,000, particularly preferably 40,000 to 50,000.

In the lactic acid-glycolic acid copolymer, a molar ratio (L:G) of a structural unit (L) derived from lactic acid relative to a structural unit (G) derived from glycolic acid is not particularly limited and can be appropriately selected depending on the purpose. However, it is preferably 1:99 to 99:1, more preferably 25:75 to 99:1, even more preferably 30:70 to 90:10, particularly preferably 50:50 to 85:15.

### <<Polyhydroxyalkanoate>>

Preferred examples of the polyhydroxyalkanoate include homopolymers and copolymers of 3-hydroxyalkanoatethat are consisting of repeating units represented by the general formula (1): -CH(R)- CH₂CO-O, where R is an alkyl group represented by -CₙH₂ₙ₊₁, and n is an integer of 1 to 15. More specifically, homopolymers and copolymers of at least one monomer selected from the group consisting of 3-hydroxypropionate, 3-hydroxybutyrate, 3-hydroxyvalerate, 3-hydroxyhexanoate, 3-hydroxyheptanoate, 3-hydroxyoctanoate, 3-hydroxynonanoate, 3-hydroxydecanoate, 3-hydroxytetradecanoate, 3-hydroxyhexadecanoate, and 3-hydroxyoctadecanoate can be used. Specific examples of the homopolymers and copolymers of 3-hydroxyalkanoate include a homopolymer of one of the above-mentioned 3-hydroxyalkanoate, a copolymer of two or more of the above-mentioned 3-hydroxyalkanoates with different n, and a mixture of two or more selected from the group consisting of the above-mentioned homopolymers and the above-mentioned copolymers. Of these, a homopolymer, a copolymer, and a mixture each composed of at least one repeating unit selected from the group consisting of a 3-hydroxybutyrate repeating unit with n=1, a 3-hydroxyvalerate repeating unit with n=2, a 3-hydroxyhexanoate repeating unit with n=3, a 3-hydroxyoctanoate repeating unit with n=5, and a 3-hydroxyoctadecanoate repeating unit with n=15 are preferable, and a copolymer composed of a 3-hydroxybutyrate repeating unit and at least one repeating unit selected from the group consisting of a 3-hydroxyvalerate repeating unit, a 3-hydroxyhexanoate repeating unit, and a 3-hydroxyoctanoate repeating unit is more preferable.

When a copolymer of 3-hydroxybutyrate and 3-hydroxyhexanoate is used, the ratio of 3-hydroxybutyrate relative to 3-hydroxyhexanoate is not particularly limited and can be appropriately selected depending on the purpose. However, as the ratio of 3-hydroxybutyrate increases, the viscosity of a particle composition liquid increases, making it difficult to eject droplets in a droplet ejection step. Thus, the ratio of 3-hydroxybutyrate relative to 3-hydroxyhexanoate is preferably in a range of 94:6 to 80:20, more preferably in a range of 90:10 to 80:20.

The weight-average molecular weight Mw of the polyhydroxyalkanoate is not particularly limited and can be appropriately selected depending on the purpose. However, as the molecular weight increases, the viscosity of the particle composition liquid increases, making it difficult to eject droplets in the droplet ejection step. Thus, the weight-average molecular weight Mw is preferably 2,000 to 1,000,000, more preferably 2,000 to 600,000.

### <Other components>

The resin particle according to embodiments of the present invention can be used in a cosmetic and the like in combination with other components such as a biologically active substance and a dispersion stabilizer as necessary. Further, the resin particle may be a functional particle depending on various applications. The functional particle is not particularly limited and can be appropriately selected depending on the purpose. Examples of the functional particle include, but are not limited to, an immediate release particle, a sustained release particle, a pH-dependent release particle, a pH-independent release particle, an enteric coated particle, a release-controlled coated particle, and a nanocrystal-containing particle.

### <<Biologically active substance>>

The biologically active substance is not particularly limited and can be appropriately selected depending on the purpose. Examples thereof include, but are not limited to, an alcohol, a fatty alcohol, or a polyol, an aldehyde, an alkanolamine, an alkoxylated alcohol (e.g., a polyethylene glycol derivative of an alcohol or a fatty alcohol), an alkoxylated amide, an alkoxylated amine, an alkoxylated carboxylic acid, an amide or a salt thereof(e.g., a ceramide or the like), an amine, an amino acid, or a salt thereof or an alkyl-substituted derivative thereof, an ester, an alkyl-substituted or an acyl derivative, a polyacrylic acid, an acrylamide copolymer, an adipic acid copolymer, an amino silicone, a biological polymer or a derivative thereof, a butylene copolymer, a carbohydrate (e.g., a polysaccharide, chitosan, a derivative thereof), a carboxylic acid, a carbomer, an ester, an ether or a polymer ether (e.g., a polyethylene glycol (PEG) derivative, a polypropylene glycol (PPG) derivative), a glyceryl ester or a derivative thereof, a halogen compound, a heterocyclic compound or a salt thereof, a hydrophilic colloid or a derivative thereof including a salt or rubber (e.g., a cellulose derivative, gelatin, xanthan gum, natural rubber), an imidazoline, an inorganic substance (e.g., clay, TiO₂, ZnO), a ketone (e.g., camphor), an isethionate, lanolin or a derivative thereof, an organic salt, a phenol or a salt thereof (e.g., a paraben), a phosphorus compound (e.g., a phosphoric acid derivative), a polyacrylate or an acrylate copolymer, a protein or an enzyme derivative (e.g., collagen), a synthetic polymer or a salt thereof, a siloxane or a silane, a sorbitan derivative, a sterol, a sulfonic acid or a derivative thereof, and wax. These may be used alone or in combination of two or more.

### <<Dispersion stabilizer>>

The dispersion stabilizer is not particularly limited. However, an alkaline earth metal salt, for example, a poorly water-soluble inorganic compound such as, for example, calcium carbonate or calcium phosphate, or the like can be used. Among the above, calcium carbonate surface-treated with a silane coupling agent is preferable because it is highly compatible with the biodegradable resin and has excellent dispersion stability. The amount of the alkaline earth metal component included in the resin particles is preferably less than 10 ppm relative to the mass of the entire resin particles from the viewpoint of preventing the aggregation of the resin particles caused by the adsorption of moisture in the air to the dispersion stabilizer, and is preferably 0.05 ppm or more to effectively exert the dispersion stabilizing effect.

Examples of a method for measuring the amount of the alkaline earth metal component included in the resin particles include the following method. First, 1.0 g of the resin particles to be measured is precisely weighed in a crucible and heated at 450°C for 3 hours using an electric furnace to incinerate the resin particles. The incinerated resin particles are dissolved in 2 ml of concentrated hydrochloric acid, and the volume is adjusted to 50 ml with ultrapure water to prepare a measurement sample. The amount of the alkaline earth metal component can be measured using a multi-type ICP emission spectrometer (manufactured by Shimadzu Corp., "ICPE-9000").

### (Method for producing resin particle)

A method for producing the resin particle according to embodiments of the present invention includes a droplet ejection step in which a particle composition liquid including a biodegradable resin and a solvent is ejected into a gas as droplets, and a granulation step in which the solvent is removed from the droplets to granulate particles, and includes other steps as necessary.

There are multiple conventionally known dry granulation methods for granulating particles in a gas.

Examples thereof include an in-gas pulverization method such as a method in which a particle material is melted and kneaded to uniformly disperse it, and the resulting melt-kneaded product is cooled and then pulverized using a pulverizer to obtain small-sized pulverized particles, and a method in which a liquid including a particle material is freeze-dried and then pulverized using a pulverizer to obtain small-sized pulverized particles.

Further, other examples include a spray drying method such as a method in which a liquid including a particle material is sprayed into a gas and dried to obtain small-sized spray particles. Note that examples of the spraying method include a pressurized nozzle method in which a liquid is pressurized and sprayed from a nozzle, and a disk method in which a liquid is sent to a high-speed rotating disk and scattered by centrifugal force.

While the in-gas pulverization method only requires simple equipment for pulverization, it is difficult to produce particles with a narrow particle size distribution by this method.

The spray drying method can produce particles that have a high ratio of the physiologically active substance retained in the particles (biologically active substance retention rate) through the particle production step. However, it is generally difficult to produce small-sized particles by this method.

If the spraying method is the disk method, it may be possible to produce small-sized particles, but the method requires large-scale equipment.

A method for producing a resin particle of the present embodiment described below does not fall in neither of the above-mentioned in-gas pulverization method nor spray drying method. The method can produce particles that have a high ratio of the physiologically active substance retained in the particles (physiologically active substance retention rate) through the particle production step and can produce small-sized particles.

### -Droplet ejection step-

A droplet ejection step is a step in which a particle composition liquid including a biodegradable resin and a solvent is ejected into a gas as droplets.

A method for ejecting droplets is not particularly limited. However, examples thereof include the following methods.
(i) A method using an ejector that ejects a pressurized liquid as a droplet from a hole provided on a flat nozzle formation surface such as an inkjet nozzle.
(ii) A method using an ejector that ejects a pressurized liquid as a droplet from a hole with an irregular shape such as an SPG membrane.
(iii) A method using an ejector that ejects a vibrated liquid as a droplet from a hole.

Examples of the ejector (iii) that uses vibration without changing the physiological activity of the physiologically active substance due to the vibration include a means that hardly causes external stress to the particle composition liquid itself, such as an ejector using a membrane vibration method, an ejector using a Rayleigh fission method, an ejector using a liquid vibration method, or an ejector using a liquid column resonance method. These ejector may further include a means for applying pressure to a liquid for ejecting the liquid. Among these means, preferred is an ejector using a liquid column resonance method, which further includes a means for applying pressure to a liquid for ejecting the liquid.

Examples of the ejector using the liquid column resonance method include, but are not limited to, an ejector that uses a method in which a standing wave is formed using liquid column resonance by applying vibration to a liquid stored in a liquid column resonance liquid chamber, and the liquid is ejected from an ejection hole formed in the amplitude direction of the standing wave in a region that forms the antinode of the standing wave.

Note that examples of the ejector using the membrane vibration method include an ejector described in Japanese Unexamined Patent Application Publication No.2008-292976 (WO2008/114655).

Examples of the ejector using the Rayleigh fission method include an ejector described in Japanese Unexamined Patent Application Publication No.2008-065009 (US2008/0063971).

Examples of the ejector using the liquid vibration method include an ejector described in Japanese Unexamined Patent Application Publication No.2010-102195 (US2010/0104970).

As an example of the droplet ejection step, the method for ejecting the particle composition liquid including the biodegradable resin and the solvent as a droplet by vibration is described again.

The ejection method by vibration is not particularly limited, but includes, for example, the following methods. Each of the methods is described below.
(a) A method using a volume changer that changes the volume of a liquid storage by using vibration.
(b) A method using a constriction generator that releases a liquid from multiple ejection holes provided in a liquid storage while applying vibration to the liquid storage, thereby causing the liquid to transition from a columnar state through a constricted state to form a droplet.
(c) A method using a nozzle vibrator that vibrates a thin film in which a nozzle is formed.

The volume changer is not particularly limited and can be selected appropriately depending on the purpose, as long as it can change the volume of the liquid storage. Examples of the volume changer include, but are not limited to, a piezoelectric element (also referred to as a "piezo element") that expands and contracts when a voltage is applied.

Examples of the constriction generator include, but are not limited to, a means using a technology described in Japanese Unexamined Patent Application Publication No.2008-065009 (corresponding to US2008/0063971). Japanese Unexamined Patent Application Publication No.2008-065009 (corresponding to US2008/0063971) describes a means for releasing a liquid from multiple nozzle holes provided in a liquid storage while applying vibration to the liquid storage using a vibrator using a piezoelectric element in contact with a part of the liquid storage, thereby causing the liquid to transition from a columnar state through a constricted state to form a droplet.

Examples of the nozzle vibrator include, but are not limited to, a means using a technology described in Japanese Unexamined Patent Application Publication No.2008-292976 (corresponding to WO2008/114655). Japanese Unexamined Patent Application Publication No.2008-292976 (corresponding to WO2008/114655) describes a means for releasing a liquid from multiple nozzle holes thereby forming a droplet, using a thin film on which multiple nozzles are formed, provided in a liquid storage, and a piezoelectric element which vibrates the thin film, disposed around a deformable region of the thin film.

The piezoelectric element is generally used as a means for generating vibration. The piezoelectric element is not particularly limited, and the shape, size, and material of the piezoelectric element can be appropriately selected. For example, a piezoelectric element used in a conventional ink-jet ejection method can be suitably used.

The shape and size of the piezoelectric element are not particularly limited and can be appropriately selected according to the shape of the ejection hole and the like.

A material of the piezoelectric element is not particularly limited and can be appropriately selected depending on the purpose. Examples thereof include, but are not limited to, a piezoelectric ceramic such as lead zirconate titanate (PZT), a piezoelectric polymer such as polyvinylidene fluoride (PVDF), and a single crystal such as quartz, LiNbO₃, LiTaO₃, or KNbO₃.

The ejection hole is not particularly limited and can be selected appropriately depending on the purpose. Examples thereof include, but are not limited to, an opening provided in a nozzle plate or the like.

The cross-sectional shape and size of the ejection hole can be appropriately selected.

The cross-sectional shape of the ejection hole is not particularly limited and can be selected appropriately depending on the purpose. Examples thereof include, but are not limited to, (1): a tapered shape in which the opening diameter reduces from the inside (liquid storage side) toward the outside (liquid ejection side), (2): a shape in which the opening diameter narrows from the inside (liquid storage side) toward the outside (liquid ejection side) while having a round shape, (3): a shape in which the opening diameter narrows with a constant nozzle angle from the inside (liquid storage side) towards the outside (liquid ejection side), and (4): a combination of the shape (1) and the shape (2). Of these, the shape (3) is preferable, as the pressure applied to the liquid in the ejection hole is maximized.

The nozzle angle in the shape (3) is not particularly limited and can be selected appropriately depending on the purpose. However, the nozzle angle is preferably 60° or more and 90° or less. When the nozzle angle is 60° or more and 90° or less, droplet ejection can be stabilized.

The size of the ejection hole is not particularly limited and can be selected appropriately depending on the purpose. However, for example, the diameter is preferably less than 1,000 µm, more preferably 1.0 µm or more and less than 1,000 µm, even more preferably 1.0 µm or more and 500 µm or less, particularly preferably 1.0 µm or more and 50 µm or less. Note that, if the shape of the ejection hole is not a perfect circle, the diameter of a perfect circle having an area equivalent to the area of the ejection hole is employed.

### <Particle composition liquid>

The liquid (i.e., particle composition liquid) ejected in the droplet ejection step includes a biodegradable resin and a solvent. However, the biodegradable resin included in the particle composition liquid can be produced from the same various materials as the biodegradable resin included in the resin particle according to embodiments of the present invention. Thus, description of the biodegradable resin is omitted, and only the solvent is described.

The content of the biodegradable resin in the particle composition liquid is not particularly limited and can be selected appropriately depending on the purpose.

Note that the particle composition liquid and the solvent are preferably substantially free of a surfactant. By being substantially free of a surfactant, safety can be improved when the particle produced is included in a pharmaceutical composition, a functional food, a functional cosmetic, or the like. Examples of the case of being substantially free of a surfactant include, but are not limited to, a case where the content of a surfactant in the particle composition liquid and in the solvent is equal to or lower than the detection limit, that is, the content cannot be detected by liquid chromatography, and a case where a surfactant is not included in the particle composition liquid and in the solvent.

### <<Solvent>>

Examples of the solvent include, but are not limited to, water, an aliphatic halogenated hydrocarbon (e.g., dichloromethane, dichloroethane, chloroform), an alcohol (e.g., methanol, ethanol, propanol, 3-methoxy-3-methyl-1-butanol, 2,2,2-trifluoroethanol), a ketone (e.g., acetone, methyl ethyl ketone), an ether (e.g., diethyl ether, dibutyl ether, 1,4-dioxane), an aliphatic hydrocarbon (e.g., n-hexane, cyclohexane, n-heptane), an aromatic hydrocarbon (e.g., benzene, toluene, xylene), an organic acid (e.g., acetic acid, propionic acid), an ester (e.g., ethyl acetate, 3-methoxy-3-methyl-1-butyl acetate), an amide (e.g., dimethylformamide, dimethylacetamide), and a mixed solvent thereof. These may be used alone or in combination of two or more. Of these, from the viewpoint of solubility, an aliphatic halogenated hydrocarbon, an alcohol, and a mixed solvent thereof are preferable, and dichloromethane, 1,4-dioxane, dimethylformamide, acetonitrile, and a mixed solvent thereof are more preferable.

The content of the solvent is preferably 70% by mass or more and 99.5% by mass or less, more preferably 90% by mass or more and 99% by mass or less, based on the mass of the particle composition liquid. When the content is 70% by mass or more and 99.5% by mass or less, production stability is improved due to the solubility of the particle material and the liquid viscosity.

The viscosity of the particle composition liquid is not particularly limited and can be selected appropriately depending on the purpose. However, the viscosity is preferably 0.5 mPa·s or more and 15.0 mPa·s or less, more preferably 0.5 mPa·s or more and 10.0 mPa·s or less. Note that the viscosity can be measured, for example, using a viscoelasticity measuring device (device name: MCR rheometer, manufactured by Anton Paar GmbH) under conditions of 25°C and a shear rate of 10 s⁻¹. The viscosity of the liquid is preferably 0.5 mPa·s or more and 15.0 mPa·s or less, which allows the above-mentioned means for ejecting a droplet to perform suitable ejection.

The surface tension of the particle composition liquid is not particularly limited and can be selected appropriately depending on the purpose. However, the surface tension is preferably 10 mN/m or more and 60 mN/m or less, more preferably 20 mN/m or more and 50 mN/m or less. Note that the surface tension can be measured, for example, using a portable surface tension meter (device name: PocketDyne, manufactured by KRUSS) by the maximum bubble pressure method under conditions of 25°C and a lifetime of 1,000 ms. The surface tension of the liquid is preferably 0.5 m Pa·s or more and 15.0 m Pa·s or less, which allows the above-mentioned means for ejecting a droplet to perform suitable ejection.

### -Granulation step-

The granulation step is a step in which the solvent is evaporated from the droplets to remove the solvent included in the droplets, thereby granulating particles.

Note that the granulation step is performed in a gas. More specifically, the granulation step is preferably performed while the droplets ejected into the gas in the droplet ejection step are flying in the gas.

The particles produced by the present method differ from those produced by a conventional spray drying method. With this method, the droplets of substantially uniform size can be ejected while being controlled to prevent coalescence of the droplets, and granulation can be achieved by evaporating the solvent from these droplets, allowing for the mass production of the uniformly sized particles with a narrow particle size distribution.

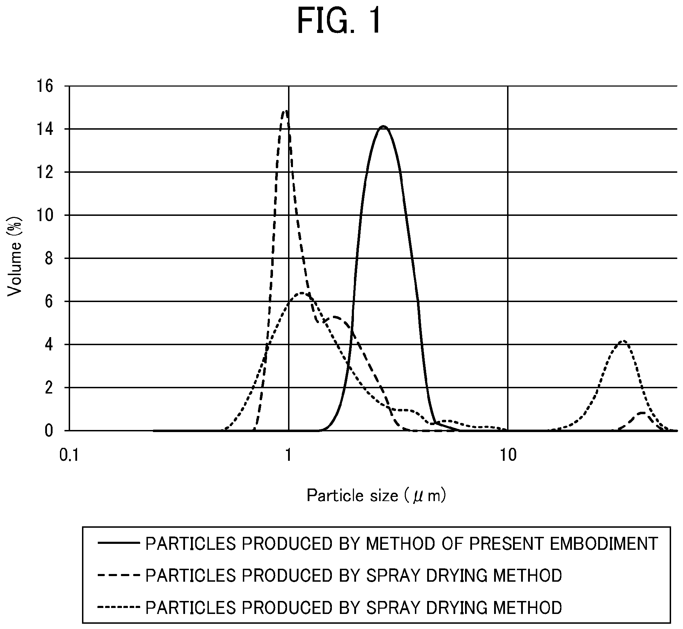

FIG. 1 is a diagram illustrating particle size distributions of particles produced by the method of the present embodiment and particles produced by a spray drying method. According to FIG. 1, unlike the particles produced by the spray drying method, the particles produced by the method of the present embodiment show only one narrow peak in the particle size distribution without any peak indicating the presence of coarse particles.

Further, the particle size can be adjusted by appropriately adjusting the size of the ejection holes of the ejector for forming the droplets.

Note that, in the granulation step, the droplets may be ejected into a transport airflow to evaporate the solvent from the droplets, thereby granulating the particles. A method for evaporating the solvent from the droplets using the transport airflow is not particularly limited and can be appropriately selected depending on the purpose. Preferable examples thereof include, but are not limited to, a method in which the transport direction of the transport airflow is set to the direction substantially perpendicular to the direction in which the droplets are ejected.

Further, it is preferable to appropriately adjust the temperature, vapor pressure, type of gas, and the like of the transport airflow. Note that a heater may be provided to adjust the temperature of the transport airflow. As described above, in the granulation step, ejection is performed while preventing the coalescence of the droplets. This makes it possible to reduce the degree of heating by the heater. Specifically, heating can be performed at a level where the biological activity of the physiologically active substance does not change.

Further, as long as the collected particles remain in a solid state, the solvent does not need to be completely evaporated, and a drying step may be added as a separate step after collection. The spraying liquid may be heated to a temperature equal to or higher than the melting point of the biodegradable resin, and after spraying, the biodegradable resin in the droplets may be solidified by the cooling effect caused by the evaporation of the solvent, thereby granulating the particles. Further, a method of evaporating the solvent from the droplets by applying a temperature change, a chemical change, or the like may be used.

### -Other steps-

The other steps are not particularly limited and can be appropriately selected depending on the purpose. Examples of thereof include, but are not limited to, a particle collecting step.

The particle collecting step is a step of collecting the produced particles and can be suitably performed by a particle collector. The particle collector is not particularly limited and can be appropriately selected depending on the purpose. Examples thereof include, but are not limited to, a cyclone collector and a back filter.

For producing the particles including at least two types of base materials, one of which is distributed more toward the surface side, such particles can be formed in the granulation step by appropriately selecting the types of base materials included in the particle composition liquid.

For forming the particles in which one of the at least two types of base materials is distributed more toward the surface side are formed in the granulation step, the contact angles of the at least two types of base materials are made different from each other. In this manner, the interaction between the base materials increases when the solvent is evaporated from the droplets in the granulation step.

In this case, since the contact angles of the at least two types of base materials are different from each other, the base materials are likely to undergo phase separation. As a result, one of the at least two types of base materials is likely to be distributed more toward the surface side. After that, as the solvent evaporates, the base materials solidify in this state to form the particles. With this method, it is possible to form the particles in which one of the at least two types of base materials is distributed more toward the surface side by only one step.

Examples of the other steps besides those described above include, but are not limited to, a step of filtering or sieving the particles after granulation to obtain the particles of uniform size and an additional drying step of removing the solvent in the particles.

Regarding the amount of the solvent included in the particles, the amount of the residual solvent in the particles is preferably less than 1% or less as measured by gas chromatography. If it is 1% or more, the particles tend to stick together, causing aggregation, which deteriorates the particle size distribution and increases the span factor. Further, the amount of the residual solvent is preferably 1 ppm or more. If it is less than 1 ppm, there is insufficient solvent in the spray liquid, and the viscosity of the spray liquid becomes high, making it difficult to form the particles.

A method for measuring the amount of the residual solvent in the particles is as follows: 2 parts by weight of 2-propanol are added to 1 part by weight of the particles to be measured, and the resulting mixture is dispersed ultrasonically for 30 minutes. The mixture is then stored in a refrigerator (5°C) for one day or more to extract the solvent in the particles. The supernatant is analyzed by gas chromatography (GC-14A, Shimadzu Corp.) to quantify the amount of the solvent in the particles.

### <Resin particle producing apparatus>

A resin particle producing apparatus includes a droplet ejector for ejecting a particle composition liquid including a biodegradable resin and a solvent into a gas as droplets, a granulator for granulating particles by removing the solvent from the droplets, and other means as necessary.

### -Droplet ejector-

The droplet ejector is a means for ejecting the particle composition liquid including the biodegradable resin and the solvent into a gas to form droplets.

In a preferable aspect, the droplet ejector ejects the particle composition liquid by vibration to form droplets.

The droplet ejector is connected to a liquid storage container described below. A connector for connecting the droplet ejector and the liquid storage container is not particularly limited and can be appropriately selected depending on the purpose, as long as the connector can supply the liquid from the liquid storage container to the droplet ejector. Examples of the connector include, but are not limited to, a tube (a pipe or the like).

The droplet ejector preferably includes a vibration imparting member that imparts vibration to the liquid to discharge a droplet. The vibration is not particularly limited and can be appropriately selected depending on the purpose. For example, the frequency is preferably 1 kHz or more, more preferably 150 kHz or more, even more preferably 300 kHz or more and 500 kHz or less. When the vibration is 1 kHz or more, a liquid column sprayed from an ejection hole can be reliably turned into a droplet. When the vibration is 150 kHz or more, production efficiency can be improved.

Examples of the droplet ejector including the vibration imparting member include, but are not limited to, an inkjet nozzle. Examples of an ejection mechanism of the inkjet nozzle which can be used include, but are not limited to, a liquid column resonance method, a membrane vibration method, a liquid vibration method, and a Rayleigh fission method.

Next, a liquid column resonance droplet ejector is specifically described as an example of the droplet ejector.

It is readily understood by those skilled in the art that the droplet ejector is not limited to a liquid column resonance droplet ejector, and other droplet ejectors (e.g., an ejector using the membrane vibration method, an ejector using the Rayleigh fission method, an ejector using the liquid vibration method, and the like) may be used.

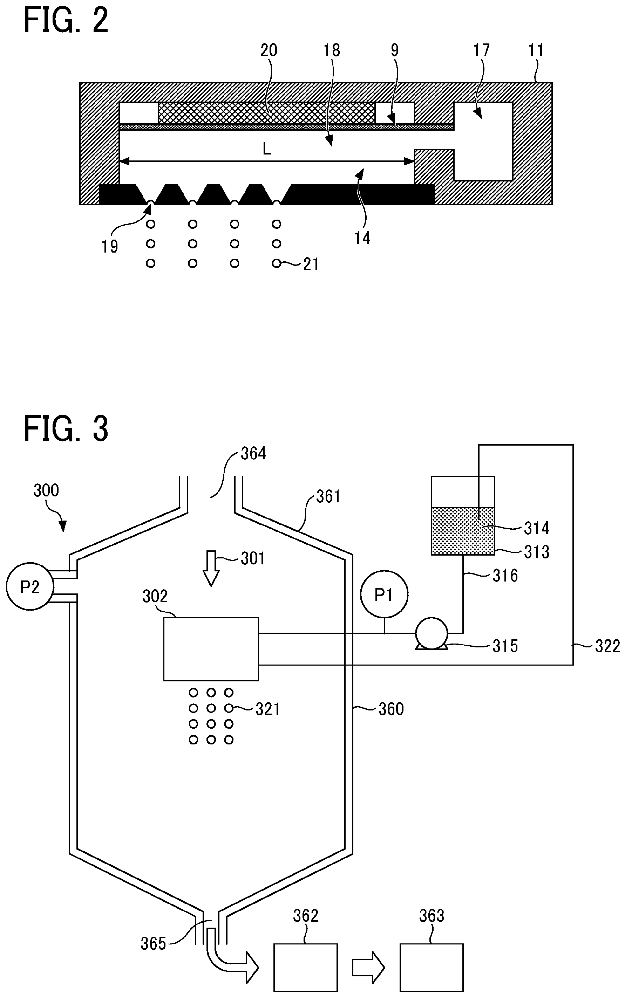

FIG. 2 is a schematic cross-sectional view illustrating a liquid column resonance droplet ejector. A liquid column resonance droplet ejector 11 includes a common liquid supply channel 17 and a liquid column resonance liquid chamber 18. The liquid column resonance liquid chamber 18 is connected to the common liquid supply channel 17 provided on one of wall surfaces at both ends in the longitudinal direction. Further, the liquid column resonance liquid chamber 18 includes an ejection hole 19 that ejects a droplet 21 on one of wall surfaces that are continuous to the wall surfaces at both ends, and a vibration generator 20 that is provided on the wall surface facing the ejection hole 19 and generates high-frequency vibration to form a liquid column resonant standing wave. Note that a high-frequency power source is connected to the vibration generator 20. Further, an airflow passage for supplying airflow that transports the droplet 21 ejected from the liquid column resonance droplet ejector 11 may be provided.

A particle composition liquid 14 including a biodegradable resin and a solvent is passed through a liquid supply pipe by a liquid circulation pump, flows into the common liquid supply channel 17 of the liquid column resonance droplet ejector 11, and is supplied to the liquid column resonance liquid chamber 18.

Then, in the liquid column resonance liquid chamber 18 filled with the particle composition liquid 14, a pressure distribution is formed by the liquid column resonance standing wave generated by the vibration generator 20. Then, the droplet 21 is ejected from the ejection hole 19 disposed in a region forming an antinode of the liquid column resonance standing wave where the amplitude is large and the pressure fluctuation is large.

The region forming the antinode of the standing wave generated by the liquid column resonance is a region other than nodes of the standing wave, and is preferably a region in which the pressure fluctuation of the standing wave has the amplitude large enough to eject the liquid, more preferably a region within ±1/4 wavelength from the position where the amplitude of the pressure standing wave is maximum (i.e., an antinode in the velocity standing wave) toward the position where the amplitude of the pressure standing wave is minimum.

Even when the multiple ejection holes are opened, the regions forming the antinodes of the standing wave make it possible to form substantially uniform droplets from each ejection hole, enabling efficient droplet ejection while reducing the likelihood of clogging of the ejection holes. Note that the particle composition liquid 14 that has passed through the common liquid supply channel 17 is circulated by a liquid return pipe. When the amount of the particle composition liquid 14 in the liquid column resonance liquid chamber 18 decreases due to the ejection of the droplet 21, a suction force caused by the action of the liquid column resonance standing wave in the liquid column resonance liquid chamber 18 acts to increase the flow rate of the particle composition liquid 14 supplied from the common liquid supply channel 17. As a result, the liquid column resonance liquid chamber 18 is replenished with the particle composition liquid 14. When the liquid column resonance liquid chamber 18 is replenished with the particle composition liquid 14, the flow rate of the particle composition liquid 14 passing through the common liquid supply channel 17 returns to the original value.

The liquid column resonance liquid chamber 18 in the liquid column resonance droplet ejector 11 is formed by joining frames formed of a material, such as metal, a ceramic, or silicone, with high rigidity that does not affect the resonant frequency of the liquid at the driving frequency.

Further, as illustrated in FIG. 2, a length L between the wall surfaces at both ends in the longitudinal direction of the liquid column resonance liquid chamber 18 is determined based on the liquid column resonance principle.

Further, for drastically improving productivity, it is preferable that a plurality of liquid column resonance liquid chambers 18 are provided for each droplet formation unit.

The number of the liquid column resonance liquid chambers 18 is not particularly limited. However, it is preferably 1 or more and 2,000 or less.

Further, a flow channel for supplying the liquid is connected to each liquid column resonance liquid chamber from the common liquid supply channel 17, and the common liquid supply channel 17 is connected to the plurality of liquid column resonance liquid chambers 18.

Further, the vibration generator 20 in the liquid column resonance droplet ejector 11 is not particularly limited as long as it can cause vibration at a predetermined frequency. However, a configuration in which a piezoelectric body is adhered to an elastic plate 9 is preferable.

From the viewpoint of productivity, the frequency is more preferably 150 kHz or more, even more preferably 300 kHz or more and 500 kHz or less.

The elastic plate 9 constitutes a part of the wall of the liquid column resonance liquid chamber so as to prevent the piezoelectric body from contacting the liquid.

Examples of the piezoelectric body include, but are not limited to, a piezoelectric ceramic such as lead zirconate titanate (PZT), which is generally used in layers due to its small displacement. Other examples include, but are not limited to, a piezoelectric polymer such as polyvinylidene fluoride (PVDF), and a single crystal such as quartz, LiNbO₃, LiTaO₃, or KNbO₃.

Further, it is preferable that the vibration generator 20 is disposed so that each liquid column resonance liquid chamber can be individually controlled.

Further, it is preferable to cut a part of the block-shaped vibration member made of one of the above-mentioned materials according to the arrangement of the liquid column resonance liquid chambers, so that each liquid column resonance liquid chamber can be individually controlled via the elastic plate.

Further, it is preferable to adopt a configuration in which the ejection holes 19 are provided in the width direction in the liquid column resonance liquid chamber 18, as this allows a large number of openings of the ejection holes 19 to be provided, thereby increasing production efficiency.

Further, since the liquid column resonance frequency varies depending on the arrangement of the openings of the ejection holes 19, it is desirable to appropriately determine the liquid column resonance frequency by checking the ejection of droplets.

### <<Particle composition liquid storage container>>

The particle composition liquid storage container is a container that stores the particle composition liquid including the biodegradable resin and the solvent.

The particle composition liquid storage container may or may not be flexible. A material of the particle composition liquid storage container is not particularly limited and can be appropriately selected depending on the purpose. It may be made of, for example, a resin or metal. The structure of the particle composition liquid storage container is not particularly limited and can be appropriately selected depending on the purpose. It may be, for example, a sealed structure or a non-sealed structure.

### -Granulator-

The granulator is a means for granulating particles by evaporating the solvent from the droplets, thereby removing the solvent included in the droplets.

Examples of the granulator include, but are not limited to, a member that forms a space for evaporating the solvent from the droplets.

The granulator preferably includes a transport airflow former for forming transport airflow.

Next, embodiments are described with reference to FIGs. 3 to 5.

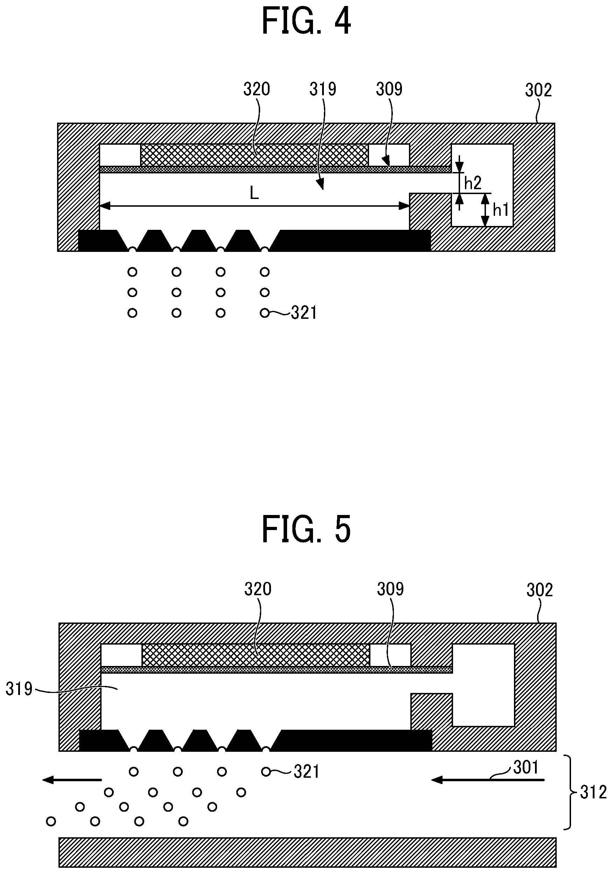

FIG. 3 is a schematic diagram illustrating a resin particle production apparatus. FIG. 4 is a schematic cross-sectional diagram illustrating a droplet ejector used in the resin particle production apparatus of FIG. 3. FIG. 5 is another schematic cross-sectional diagram illustrating a droplet ejector used in the resin particle production apparatus of FIG. 3.

A resin particle production apparatus 300 illustrated in FIG. 3 includes a droplet ejector 302, a drying and collecting unit 360, a transport airflow outlet 365, and a particle storage 363. The droplet ejector 302 is connected to a particle composition liquid storage container 313 that stores a particle composition liquid 314, and a liquid circulation pump 315 that supplies the particle composition liquid 314 stored in the particle composition liquid storage container 313 to the droplet ejector 302 through a liquid supply pipe 316 and also pressurizes the particle composition liquid 314 in the liquid supply pipe 316 to return it to the particle composition liquid storage container 313 through a liquid return pipe 322, so that the particle composition liquid 314 can be supplied to the droplet ejector 302 at any time. A pressure measuring device P1 is provided in the liquid supply pipe 316, and a pressure measuring device P2 is provided in the drying and collecting unit 360, and a liquid sending pressure to the droplet ejector 302 and a pressure inside the drying and collecting unit 360 are controlled by the pressure measuring devices P1 and P2, respectively. In this case, if the pressure measurement value of P1 is greater than the pressure measurement value of P2, there is a risk that the particle composition liquid 314 seeps out from the ejection hole, and if the pressure measurement value of P1 is smaller than the pressure measurement value of P2, there is a risk that gas enters the droplet ejector 302, stopping the ejection. Thus, it is preferable that the pressure measurement value of P1 is approximately the same as the pressure measurement value of P2.

In a chamber 361, a downward airflow (transport airflow) 301 is provided from a transport airflow inlet 364. A droplet 321 ejected from the droplet ejector 302 is transported downward not only by gravity but also by the transport airflow 301, passes through the transport airflow outlet 365, is collected by a particle collector 362, and is stored in the particle storage 363.

Note that, in the droplet ejection step, if the ejected droplets contact each other before drying, the droplets may coalesce with each other. For obtaining particles with a narrow particle size distribution, it is preferable that a distance between the ejected droplets is maintained. However, although the ejected droplets have a certain initial speed, they eventually decelerate due to air resistance. When the droplets are not sufficiently dried, the droplets ejected later may catch up and coalesce with the decelerated droplets. To prevent the coalescence, it is preferable to dry the droplets and transport them while preventing the droplets from decelerating and contacting each other by the transport airflow 301, thereby preventing the coalescence of the droplets. Thus, it is preferable to provide the transport airflow 301 in the same direction as the droplet ejection direction near the droplet ejector 302. Note that even if the droplets contact each other, they do not coalesce with each other if they are sufficiently dry before they come into contact. In such a case, the transport airflow 301 does not need to be used.

FIG. 4 is an enlarged view of the droplet ejector 302 of the resin particle production apparatus in FIG. 3. As illustrated in FIG. 4, the droplet ejector 302 includes a volume changer 320, an elastic plate 309, and a particle composition liquid storage 319. When a voltage is applied to the volume changer 320, the droplet ejector 302 deforms and reduces the volume of the particle composition liquid storage 319, so that the particle composition liquid stored in the particle composition liquid storage 319 is ejected from the ejection hole as a droplet 321.

FIG. 5 is another diagram illustrating the droplet ejector 302 of the resin particle production apparatus in FIG. 3. As illustrated in FIG. 5, in an airflow passage 312, the transport airflow 301 may be substantially perpendicular to the ejection direction. Note that the transport airflow 301 may have an angle and is preferably at an angle causing the droplet to leave the droplet ejector 302. As illustrated in FIG. 5, when the droplets 321 are ejected by changing the volume of the particle composition liquid storage 319 via the elastic plate 309 using the volume changer 320, and the transport airflow 301 for preventing the coalescence is applied in the direction substantially perpendicularly to the ejected droplets 321, it is preferable to dispose the ejection holes so that the trajectories of the droplets do not overlap with each other when the droplets 321 are transported from the ejection holes by the transport airflow 301 for preventing the coalescence.

Note that, after the particles are prevented from the coalescence by the transport airflow 301, the particles may be transported to the particle collector by another airflow.

The speed of the transport airflow is preferably equal to or greater than the droplet ejection speed. When the speed of the transport airflow is greater than the droplet ejection speed, the droplets can be prevented from coalescing with each other.

Further, the transport airflow may be mixed with a chemical substance that promotes drying of the droplets. The state of the transport airflow is not limited and may be a laminar flow, a swirling flow, or a turbulent flow. A type of gas constituting the transport airflow is not particularly limited and may be appropriately selected depending on the purpose. The air or non-flammable gas such as nitrogen may be used.

Further, the temperature of the transport airflow can be adjusted as appropriate. However, the temperature is set such that the physiological activity of the physiologically active substance included in the droplets does not change due to the airflow temperature.

When the amount of the residual solvent included in the particles collected by the particle collector 362 illustrated in FIG. 3 is large, it is preferable to perform secondary drying as necessary to reduce the amount of the residual solvent. In the secondary drying, a commonly known dryer such as fluidized bed dryer or vacuum dryer can be used.

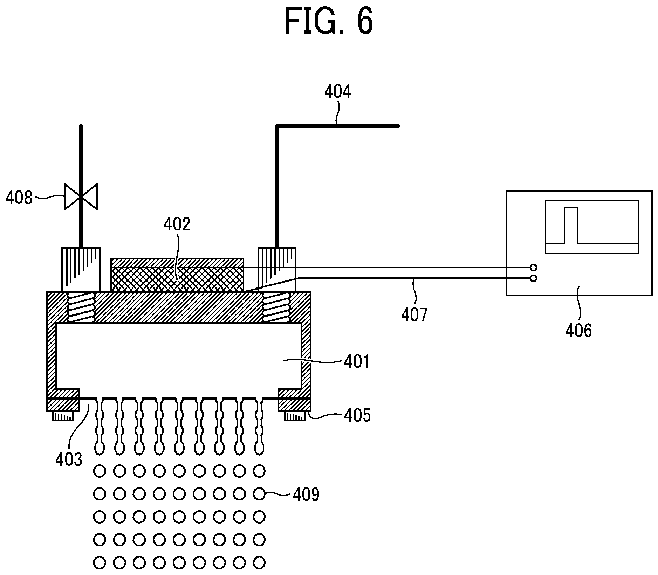

Further, as an example of the droplet ejector, an ejector using the Rayleigh fission method is described with reference to FIG. 6.

FIG. 6 is a schematic cross-sectional diagram illustrating a Rayleigh fission droplet ejector. As a preferable example, the droplet ejector includes, as presented in FIG. 6, at least a storage 401 for storing the particle composition liquid, a vibrator 402, and a plurality of through holes 403.

The storage 401 at least needs to hold the particle composition liquid in a pressurized state. Thus, it is preferable that the storage 401 is a member that is made of metal such as SUS or aluminum and is pressure-resistant to about 10 MPa, without being limited thereto. Also, for example, as illustrated in FIG. 6, the storage 401 preferably has a structure in which the storage 401 is connected to a supply pipe 404 that supplies the liquid to the storage 401 and is provided with a through-hole holding mechanism 405 that holds a plate having the through holes 403 for ejecting a droplet 409. Further, the vibrator 402 for vibrating the entire storage 401 is in contact with the storage 401. It is preferable that the vibrator 402 is connected to a vibration generator 406 by a conductive wire 407 and controlled thereby.

For ensuring stable formation of a liquid column, it is preferable to provide, for example, a release valve 408 for adjusting the pressure inside the storage 401 and removing an internal air bubble.

As the vibrator 402, one vibrator is preferably used to vibrate the entire storage 401. The vibrator 402 for applying vibration to the storage 401 is not particularly limited and can be appropriately selected and used, as long as it can reliably cause vibration at a constant frequency. However, from the above-mentioned viewpoint, it is preferable that, for example, the through holes 403 are vibrated at a constant frequency by expansion and contraction of the piezoelectric body.

The piezoelectric body has a function of converting electrical energy into mechanical energy. Specifically, when a voltage is applied to the piezoelectric body, the piezoelectric body expands and contracts, making it possible to vibrate the through holes 403.

Examples of the piezoelectric body include a piezoelectric ceramic such as lead zirconate titanate (PZT), which is generally used in layers because of their small displacement. Other examples include, but are not limited to, a piezoelectric polymer such as polyvinylidene fluoride (PVDF) and a single crystal such as quartz, LiNbO₃, LiTaO₃, or KNbO₃.

The constant frequency is not particularly limited and can be appropriately selected depending on the purpose. However, it is preferably 10 kHz to 10 MHz, more preferably 50 kHz to 2 MHz from the viewpoint of generating a minute droplet having an extremely uniform particle size.

### [Examples]

Examples of the present invention are described below. However, the present invention is not limited to these examples.

### (Example 1)

### -Preparation of formulation liquid A-

Polylactic acid (PLA-R-001F-SOR, manufactured by Nagase & Co., Ltd.) was dissolved in dichloromethane as a solvent to prepare a formulation liquid A.

### -Granulation of particle 1 (Rayleigh fission)-

The obtained formulation liquid A was ejected from the ejection holes using a Rayleigh fission droplet ejector illustrated in FIG. 6 to form droplets, and the solvent was removed from the droplets using the particle production apparatus illustrated in FIG. 3 to granulate particles 1. After granulation, the particles were vacuum dried.

The volume average particle size (Dv) of the obtained particles 1 was 16.2 µm, and the (R.S.F.) was 0.72. Note that these were measured using a laser diffraction/scattering type particle size distribution measuring device (device name: LA-960, manufactured by Horiba, Ltd.).

The average circularity was 0.966. This was measured using a flow particle image analyzer (product name "FPIA (registered trademark)-3000S", manufactured by Sysmex Corp.).

The amount of alkaline earth metals was 0.1 ppm. This was measured using a multi-type ICP emission spectrophotometer (manufactured by Shimadzu Corp., "ICPE-9000").

The solvent content of the particles was 0.9%. The solvent content of the particles was 0.9%. This was measured using a gas chromatograph (GC-14A, Shimadzu Corp.).

Note that the particle production conditions are as follows.

### [Particle production conditions]

· Shape of ejection hole: perfect circle
· Diameter of ejection hole: 10 µm
· Extrusion pressure of formulation liquid: 0.4 MPa
· Excitation frequency: 150 kHz
· Excitation voltage: 5 V
· Transport airflow rate: 50 m³/h
· Drying airflow temperature: 40°C
· Vacuum drying after granulation: pressure 500 Pa, temperature 30°C, drying time 24 hours

### (Example 2)

### -Preparation of formulation liquid B-

Polylactic-glycolic acid (PDLG5004, manufactured by Corbion Japan K.K.) was dissolved in a mixed solvent of acetonitrile and water in a mass ratio of 9:1 as a solvent to prepare a formulation liquid B.

### -Granulation of particle 2 (Rayleigh fission)-

Particles 2 were granulated in the same manner as in Example 1 using the obtained formulation liquid B, except that the particle production conditions were changed as follows. The volume average particle size (Dv) of the obtained particles 2 was 26.9 µm, and the (R.S.F.) was 0.52. The average circularity was 0.981. The amount of alkaline earth metals was 0.1 ppm. The solvent content of the particles was 0.5%.

### [Particle production conditions]

· Shape of ejection hole: perfect circle
· Diameter of ejection hole: 30 µm
· Extrusion pressure of formulation liquid: 0.18 MPa
· Excitation frequency: 80 kHz
· Excitation voltage: 5 V
· Transport airflow rate: 50 m³/h
· Drying airflow temperature: 50°C
· Vacuum drying after granulation: pressure 500 Pa, temperature 30°C, drying time 24 hours

### (Example 3)

### -Preparation of formulation liquid C-

Polyhydroxyalkanoate (BP350-15, 3-hydroxybutyrate:3-hydroxyhexanoate ratio = 90:10, Blupha Co., Ltd.) was dissolved in a mixed solvent of dichloromethane and ethanol in a mass ratio of 6:4 as a solvent to prepare a formulation liquid C.

### -Granulation of particle 3 (Rayleigh fission)-

Particles 3 were granulated in the same manner as in Example 1 using the obtained formulation liquid C, except that the particle production conditions were changed as follows. The volume average particle size (Dv) of the obtained particles 3 was 22.8 µm, and the (R.S.F.) was 0.98. The average circularity was 0.925. The amount of alkaline earth metals was 0.3 ppm. The solvent content of the particles was 0.4%.

### [Particle production conditions]

· Shape of ejection hole: perfect circle
· Diameter of ejection hole: 10 µm
· Extrusion pressure of formulation liquid: 0.4 MPa
· Excitation frequency: 150 kHz
· Excitation voltage: 5 V
· Transport airflow rate: 50 m³/h
· Drying airflow temperature: 25°C
· Vacuum drying after granulation: pressure 500 Pa, temperature 30°C, drying time 24 hours

### (Example 4)

### -Preparation of formulation liquid D-

Polyhydroxyalkanoate (BP350-15, Blupha Co., Ltd.) was dissolved in a mixed solvent of dimethylformamide and acetone in a mass ratio of 5:5 as a solvent to prepare a formulation liquid D.

### -Granulation of particle 4 (Rayleigh fission)-

Particles 4 were granulated in the same manner as in Example 1 using the obtained formulation liquid D, except that the particle production conditions were changed as follows. The volume average particle size (Dv) of the obtained particles 4 was 18.9 µm, and the (R.S.F.) was 0.75. The average circularity was 0.985. The amount of alkaline earth metals was 0.3 ppm. The solvent content of the particles was 0.4%.

### [Particle production conditions]

· Shape of ejection hole: perfect circle
· Diameter of ejection hole: 15 µm
· Extrusion pressure of formulation liquid: 0.15 MPa
· Excitation frequency: 130 kHz
· Excitation voltage: 5 V
· Transport airflow rate: 50 m³/h
· Drying airflow temperature: 50°C
· Vacuum drying after granulation: pressure 500 Pa, temperature 30°C, drying time 24 hours

### (Example 5)

### -Preparation of formulation liquid E-

Polyhydroxyalkanoate (PHBV, Sigma-Aldrich) was dissolved in dimethylformamide as a solvent to prepare a formulation liquid E.

### -Granulation of particle 5 (Rayleigh fission)-

Particles 5 were granulated in the same manner as in Example 1 using the obtained formulation liquid E, except that the particle production conditions were changed as follows. The volume average particle size (Dv) of the obtained particles 5 was 20.9 µm, and the (R.S.F.) was 1.12. The average circularity was 0.982. The amount of alkaline earth metals was 0.2 ppm. The solvent content of the particles was 0.8%.

### [Particle production conditions]

· Shape of ejection hole: perfect circle
· Diameter of ejection hole: 15 µm
· Extrusion pressure of formulation liquid: 0.15 MPa
· Excitation frequency: 130 kHz
· Excitation voltage: 5 V
· Transport airflow rate: 50 m³/h
· Drying airflow temperature: 120°C
· Vacuum drying after granulation: pressure 500 Pa, temperature 30°C, drying time 24 hours

### (Example 6)

### -Preparation of formulation liquid F-

Polyhydroxyalkanoate (BP350-05, Blupha Co., Ltd.) was dissolved in a mixed solvent of dimethylformamide and acetone in a mass ratio of 5:5 as a solvent to prepare a formulation liquid F.

### -Granulation of particle 6 (Rayleigh fission)-

Particles 6 were granulated in the same manner as in Example 1 using the obtained formulation liquid F, except that the particle production conditions were changed as follows. The volume average particle size (Dv) of the obtained particles 6 was 21.9 µm, and the (R.S.F.) was 0.91. The average circularity was 0.985. The amount of alkaline earth metals was 0.1 ppm. The solvent content of the particles was 0.8%.

### [Particle production conditions]

· Shape of ejection hole: perfect circle
· Diameter of ejection hole: 15 µm
· Extrusion pressure of formulation liquid: 0.15 MPa
· Excitation frequency: 130 kHz
· Excitation voltage: 5 V
· Transport airflow rate: 50 m³/h
· Drying airflow temperature: 50°C
· Vacuum drying after granulation: pressure 500 Pa, temperature 30°C, drying time 24 hours

### (Comparative example 1)

### -Granulation of particle 7 (spray drying)-

Particles 7 were granulated using the formulation liquid D prepared in Example 4 by a spray dryer (rotary disk atomizer, manufactured by Ohkawara Kakohki Co., Ltd.) under the following particle production conditions. The volume average particle size (Dv) of the obtained particles 7 was 20.9 µm, and the (R.S.F.) was 1.54. The average circularity was 0.981. The amount of alkaline earth metals was 0.2 ppm. The solvent content of the particles was 0.8%.

### [Particle production conditions]

· Spray dryer model: L-8
· Rotary disk atomizer rotation speed: 10,000 rpm
· Formulation liquid feed rate: 2 kg/hour
· Transport airflow rate: 50 m³/h
· Drying airflow temperature: 50°C
· Vacuum drying after granulation: pressure 500 Pa, temperature 30°C, drying time 24 hours

### (Comparative example 2)

### -Granulation of particle 8 (spray drying)-

Particles 8 were granulated using the formulation liquid C prepared in Example 3 by the spray dryer (rotary disk atomizer, manufactured by Ohkawara Kakohki Co., Ltd.) under the following particle production conditions. The volume average particle size (Dv) of the obtained particles 8 was 19.8 µm, and the (R.S.F.) was 1.51. The average circularity was 0.932. The amount of alkaline earth metals was 0.2 ppm. The solvent content of the particles was 0.3%.

### [Particle production conditions]

· Spray dryer model: L-8
· Rotary disk atomizer rotation speed: 10,000 rpm
· Formulation liquid feed rate: 2 kg/hour
· Transport airflow rate: 50 m³/h
· Drying airflow temperature: 50°C
· Vacuum drying after granulation: pressure 500 Pa, temperature 30°C, drying time 24 hours

### (Comparative example 3)

### -Preparation of formulation liquid G-

In a quartz glass pot mill, polyhydroxyalkanoate (BP350-15, Blupha Co., Ltd.) was dissolved in a mixed solvent of dimethylformamide and acetone in a mass ratio of 5:5 as a solvent. Further, a dispersion stabilizer (manufactured by Shiraishi Calcium Co., Ltd., ACTIFO RT700, amount of 20 ppm relative to the resin) and 5 mm zirconia beads were added to the mixture. The resulting mixture was treated on a ball mill rotating stand at a peripheral speed of 100 rpm for 24 hours to prepare a formulation liquid G.

### -Granulation of particle 9 (Rayleigh fission)-

Particles 9 were granulated in the same manner as in Example 1 using the obtained formulation liquid G, except that the particle production conditions were changed as follows. The volume average particle size (Dv) of the obtained particles 9 was 19.2 µm, and the (R.S.F.) was 1.31. The average circularity was 0.985. The amount of alkaline earth metals was 20 ppm. The solvent content of the particles was 0.3%.

### [Particle production conditions]

· Shape of ejection hole: perfect circle
· Diameter of ejection hole: 15 µm
· Extrusion pressure of formulation liquid: 0.15 MPa
· Excitation frequency: 130 kHz
· Excitation voltage: 5 V
· Transport airflow rate: 50 m³/h
· Drying airflow temperature: 50°C
· Vacuum drying after granulation: pressure 500 Pa, temperature 30°C, drying time 24 hours

### (Comparative example 4)

### -Granulation of Particle 10 (Rayleigh fission)-

Particles 10 were granulated in the same manner as in Example 1 using the formulation liquid D prepared in Example 4, except that the particle production conditions were changed as follows. The volume average particle size (Dv) of the obtained particles 10 was 18.1 µm, and the (R.S.F.) was 1.62. The average circularity was 0.924.

The amount of alkaline earth metals was 0.3 ppm. The solvent content of the particles was 2.3%.

### [Particle production conditions]

· Shape of ejection hole: perfect circle
· Diameter of ejection hole: 15 µm
· Extrusion pressure of formulation liquid: 0.15 MPa
· Excitation frequency: 130 kHz
· Excitation voltage: 5 V
· Transport airflow rate: 50 m³/h
· Drying airflow temperature: 50°C
· Vacuum drying after granulation: not performed

### (Example 7)

### -Preparation of formulation liquid H-

Polybutylene adipate terephthalate (TH801T, Chori Co., Ltd.) was dissolved in dichloromethane as a solvent to prepare a formulation liquid H.

### -Granulation of particle 11 (Rayleigh fission)-

Particles 11 were granulated in the same manner as in Example 1 using the obtained formulation liquid H, except that the particle production conditions were changed as follows. The volume average particle size (Dv) of the obtained particles 11 was 19.9 µm, and the (R.S.F.) was 0.89. The average circularity was 0.991. The amount of alkaline earth metals was 0.1 ppm. The solvent content of the particles was 0.7%.

### [Particle production conditions]

· Shape of ejection hole: perfect circle
· Diameter of ejection hole: 15 µm
· Extrusion pressure of formulation liquid: 0.15 MPa
· Excitation frequency: 130 kHz
· Excitation voltage: 5 V
· Transport airflow rate: 50 m³/h
· Drying airflow temperature: 50°C
· Vacuum drying after granulation: pressure 500 Pa, temperature 30°C, drying time 24 hours

### (Example 8)

### -Preparation of formulation liquid I-

Polyhydroxyalkanoate (BP350-15, Blupha Co., Ltd.) was mixed with dimethylformamide as a solvent. For adjusting the molecular weight of the polyhydroxyalkanoate by thermal decomposition, the mixture was heated to 150°C, stirred for 15 minutes, and then cooled to room temperature. Acetone was added to this solution to prepare a formulation liquid I with a mass ratio of dimethylformamide relative to acetone of 5:5.

### -Granulation of particle 12 (Rayleigh fission)-

Particles 12 were granulated in the same manner as in Example 1 using the obtained formulation liquid I, except that the particle production conditions were changed as follows. The volume average particle size (Dv) of the obtained particles 12 was 24.4 µm, and the (R.S.F.) was 0.89. The average circularity was 0.922. The amount of alkaline earth metals was 0.2 ppm. The solvent content of the particles was 0.5%.

### [Particle production conditions]

· Shape of ejection hole: perfect circle
· Diameter of ejection hole: 15 µm
· Extrusion pressure of formulation liquid: 0.15 MPa
· Excitation frequency: 130 kHz
· Excitation voltage: 5 V
· Transport airflow rate: 50 m³/h
· Drying airflow temperature: 50°C
· Vacuum drying after granulation: pressure 500 Pa, temperature 30°C, drying time 24 hours

Table 1 shows the production conditions for the particles 1 to 12. Note that, in Table 1, the term "PLA" stands for "polylactic acid". The term "PLGA" stands for "poly(lactic-co-glycolic acid)". The term "PHBH" stands for "poly(3-hydroxybutyrate-co-3-hydroxyhexanoate)". The term "PHBV" stands for "poly(3-hydroxybutyrate-co-3-hydroxyvalerate)".

The term "PBAT" stands for "polybutylene adipate terephthalate".

**Table 1**

| | | Biodegradable resins | | Solvents | | | Granulation methods |
|---|---|---|---|---|---|---|---|
| | | Type | Mw | Solvent A | Solvent B | A:B | |
| Example 1 | Particle 1 | PLA | 63,000 | CH₂Cl₂ | None | - | Rayleigh fission method |
| Example 2 | Particle 2 | PLGA | 44,000 | MeCN | H₂O | 9:1 | Rayleigh fission method |
| Example 3 | Particle 3 | PHBH | 326,000 | CH₂Cl₂ | EtOH | 6:4 | Rayleigh fission method |
| Example 4 | Particle 4 | PHBH | 326,000 | DMF | Acetone | 5:5 | Rayleigh fission method |
| Example 5 | Particle 5 | PHBV | 716,000 | DMF | None | - | Rayleigh fission method |
| Example 6 | Particle 6 | PHBH | 413,000 | DMF | Acetone | 5:5 | Rayleigh fission method |
| Example 7 | Particle 11 | PBAT | 106,293 | CH₂Cl₂ | None | - | Rayleigh fission method |
| Example 8 | Particle 12 | PHBH | 45,000 | DMF | Acetone | 5:5 | Rayleigh fission method |
| Comparative example 1 | Particle 7 | PHBH | 326,000 | DMF | Acetone | 5:5 | Spray drying method |
| Comparative example 2 | Particle 8 | PHBH | 326,000 | CH₂Cl₂ | EtOH | 6:4 | Spray drying method |
| Comparative example 3 | Particle 9 | PHBH | 326,000 | DMF | Acetone | 5:5 | Rayleigh fission method |
| Comparative example 4 | Particle 10 | PHBH | 326,000 | DMF | Acetone | 5:5 | Rayleigh fission method |

### (Production of powder foundation)

10 parts by mass of each of the resin particle groups of Examples 1 to 8 and Comparative examples 1 to 4, and 21 parts by mass of sericite, 56 parts by mass of muscovite, 0.6 parts by mass of red iron oxide, 1 part by mass of yellow iron oxide, and 0.1 parts by mass of black iron oxide as pigments were mixed using a Henschel mixer to produce a mixture. On the other hand, 10 parts by mass of 2-ethylhexanoic acid cetyl ester as an oily base material, 1 part by mass of sorbitan sesquioleate as an emulsifier, and 0.2 parts by mass of a preservative were mixed and dissolved to produce a solution. After the above mixture and the above solution were uniformly mixed, 0.1 parts by mass of a fragrance were added and mixed uniformly, and then the resulting mixture was crushed and passed through a sieve to produce a foundation material. This foundation material was compression molded into a metal plate to produce a powder foundation. Ten panelists spread the obtained powder foundation on their wrists and evaluated the powder foundation for adhesion to the skin and soft spreadability on the skin according to the following criteria. The results are shown in Table 2. Note that the numerical values of the evaluation results in the table are the average values of the test results of 10 panelists, and a score of 4.0 or higher was considered acceptable.

### [Evaluation criteria]

5: Very good
4: Good
3: Fairly good
2: Not very good
1: Not good

**Table 2**

| | Particles | | | | | | Evaluation results | |
|---|---|---|---|---|---|---|---|---|
| | Type | Volume average particle size Dv (µm) | R.S.F. | Average circularity | Alkaline earth metal content (ppm) | Solvent content (%) | Adhesion | Soft spreadability |
| Example 1 | Particle 1 | 16.2 | 0.72 | 0.966 | 0.1 | 0.9 | 4.3 | 4.1 |
| Example 2 | Particle 2 | 26.9 | 0.52 | 0.981 | 0.1 | 0.5 | 4.5 | 4.2 |
| Example 3 | Particle 3 | 22.8 | 0.98 | 0.925 | 0.3 | 0.4 | 4.2 | 4.1 |
| Example 4 | Particle 4 | 18.9 | 0.75 | 0.985 | 0.3 | 0.4 | 4.3 | 4.3 |
| Example 5 | Particle 5 | 20.9 | 1.12 | 0.982 | 0.2 | 0.8 | 4.0 | 4.0 |
| Example 6 | Particle 6 | 21.9 | 0.91 | 0.985 | 0.1 | 0.8 | 4.1 | 4.3 |
| Example 7 | Particle 11 | 19.9 | 0.89 | 0.991 | 0.1 | 0.7 | 4.2 | 4.4 |
| Example 8 | Particle 12 | 24.4 | 0.76 | 0.922 | 0.2 | 0.5 | 4.4 | 4.2 |
| Comparative example 1 | Particle 7 | 20.9 | 1.54 | 0.981 | 0.2 | 0.8 | 2.9 | 3.9 |
| Comparative example 2 | Particle 8 | 19.8 | 1.51 | 0.932 | 0.2 | 0.3 | 2.7 | 3.2 |
| Comparative example 3 | Particle 9 | 19.2 | 1.31 | 0.985 | 20 | 0.3 | 2.8 | 3.6 |
| Comparative example 4 | Particle 10 | 18.1 | 1.62 | 0.924 | 0.3 | 2.3 | 2.6 | 3.2 |

From the results of Examples 1 to 8, it is found that when the resin particles having a volume average particles size of 5 µm or more and 50 µm or less and a relative span factor (R.S.F) of 1.2 or less are used, a cosmetic product excellent in the adhesion to the skin and the soft use feeling on the skin can be obtained.

On the other hand, from the results of Comparative examples 1 to 4, it is found that when the (R.S.F) exceeds 1.2, at least one of the adhesion to the skin and the soft use feeling on the skin is not excellent, and such a product is unsuitable as a cosmetic.

Note that aspects of the present invention are, for example, as follows.

### <Aspect 1>

A resin particle including a biodegradable resin, in which the resin particle has a volume average particle size of 5 µm or more and 50 µm or less and a relative span factor (R.S.F) of 1.2 or less.

### <Aspect 2>

The resin particle described in the <Aspect 1>, in which the resin particle has an average circularity of greater than 0.980.

### <Aspect 3>

The resin particle described in the <Aspect 1> or <Aspect 2>, in which the biodegradable resin includes at least one of a polylactic acid and a polylactic acid-glycolic acid copolymer.

### <Aspect 4>

The resin particle described in any one of the <Aspect 1> to <Aspect 3>, in which the biodegradable resin includes a polyhydroxyalkanoate.

### <Aspect 5>

The resin particle described in the <Aspect 4>, in which the polyhydroxyalkanoate is poly(3-hydroxybutyrate-co-3-hydroxyhexanoate).

### <Aspect 6>

The resin particle described in any one of the <Aspect 1> to <Aspect 5>, in which the biodegradable resin includes polybutylene adipate terephthalate.

### <Aspect 7>

The resin particle described in any one of the <Aspect 1> to <Aspect 6>, in which the biodegradable resin has a weight-average molecular weight of 500,000 or less.

### <Aspect 8>

The resin particle described in any one of the <Aspect 1> to <Aspect 7>, in which the biodegradable resin has the weight-average molecular weight of 50,000 or less.

### <Aspect 9>

The resin particle described in any one of the <Aspect 1> to <Aspect 8>, in which the resin particle includes an alkaline earth metal component in an amount of 0.05 ppm or more and less than 10 ppm relative to a total mass of the resin particle.

### <Aspect 10>

The resin particle described in any one of the <Aspect 1> to <Aspect 9>, in which the resin particle includes an organic solvent in an amount of 1 ppm or more and less than 1% relative to a total mass of the resin particle.

### <Aspect 11>

A method for producing the resin particle described in any one of the <Aspect 1> to <Aspect 10>, including:
ejecting a particle composition liquid including the biodegradable resin and a solvent into a gas as droplets, and;
removing the solvent from the droplets to granulate particles.

### <Aspect 12>

The method described in the <Aspect 11>, in which the ejecting includes:
applying vibration to the particle composition liquid to thereby eject the particle composition liquid from ejection holes.

The resin particle described in any one of the <Aspect 1> to <Aspect 10> and the method for producing the resin particle described in the <Aspect 11> or <Aspect 12> can solve the conventional various problems and achieve the object of the present invention.

Any one of the above-described operations may be performed in various other ways, for example, in an order different from the one described above.

## Claims

1. A resin particle comprising:
a biodegradable resin,
wherein the resin particle has a volume average particle size of 5 µm or more and 50 µm or less and a relative span factor of 1.2 or less.

2. The resin particle according to claim 1, wherein the resin particle has an average circularity of greater than 0.980.

3. The resin particle according to claim 1 or 2, wherein the biodegradable resin includes at least one of a polylactic acid and a polylactic acid-glycolic acid copolymer.

4. The resin particle according to any one of claims 1 to 3, wherein the biodegradable resin includes a polyhydroxyalkanoate.

5. The resin particle according to claim 4, wherein the polyhydroxyalkanoate is poly(3-hydroxybutyrate-co-3-hydroxyhexanoate).

6. The resin particle according to any one of claims 1 to 5, wherein the biodegradable resin includes polybutylene adipate terephthalate.

7. The resin particle according to any one of claims 1 to 6, wherein the biodegradable resin has a weight-average molecular weight of 500,000 or less.

8. The resin particle according to any one of claims 1 to 7, wherein the biodegradable resin has a weight-average molecular weight of 50,000 or less.

9. The resin particle according to any one of claims 1 to 8, wherein the resin particle includes an alkaline earth metal component in an amount of 0.05 ppm or more and less than 10 ppm relative to a total mass of the resin particle.

10. The resin particle according to any one of claims 1 to 9, wherein the resin particle includes an organic solvent in an amount of 1 ppm or more and less than 1% relative to a total mass of the resin particle.

11. A method for producing the resin particle according to any one of claims 1 to 10, the method comprising:
ejecting a particle composition liquid including the biodegradable resin and a solvent into a gas as droplets; and
removing the solvent from the droplets to granulate particles.

12. The method according to claim 11, wherein the ejecting includes:
applying vibration to the particle composition liquid to thereby eject the particle composition liquid from ejection holes.
